# EUROPEAN PATENT APPLICATION

(11) **EP 1 591 118 A1**
(43) Date of publication of application: **02.11.2005**
(21) Application number: 04010013.3
(22) Date of filing: 27.04.2004
(51) Int. Cl.: A61K 31/4045, A61P 41/00, A61P 17/00, A61P 13/00, A61P 11/00

(54) **Use of melatonin in preventing postoperative complications**

(71) Applicant: Nutri-Fit GmbH & Co. KG, 49439 Mühlen (DE)
(72) Inventor: Schneider, Heinz, 1792 Cordast (CH)
(74) Representative: Wichmann, Hendrik

(57) **Abstract**

The invention relates to the use of melatonin, either alone or in combination with at least one compound selected from the group consisting of L-arginine, a physiologically acceptable salt thereof, one or more other physiologically acceptable compounds associated with the synthesis of nitric oxide and mixtures thereof, for manufacture of a medicament for the therapeutic treatment, prophylactic treatment and/or prevention of postoperative infectious and/or non-infections complications induced by surgical interventions like pneumonia, wound infection (wound dehiscence), intra-abdominal abscess, and urinary tract infection (UTI) or wherein the non-infection complication is anastomotic leak, a pharmaceutical formulation comprising melatonin and at least one compound selected from the group consisting of L-arginine, a physiologically acceptable salt thereof, one or more other physiologically acceptable compounds associated with the synthesis of nitric oxide and mixtures thereof.

## Description

The present invention relates to the use of melatonin for the preparation of a medicament which may be therapeutically administered to patients prior to surgery in order to prevent or prophylactically treat postoperative infectious and non-infectious complications induced by major surgical interventions covering both, elective as well as emergency procedures.

Melatonin (N-acetyl-5-methoxytryptamin) is a hormonal product almost exclusively produced and secreted by the pineal gland. Melatonin is involved in circadian rythms and the sleep-wake cycle of vertebrates. Besides its main function of sleep induction, melatonin is also known for its immune modulating activities as well as for its antioxidative effects. As a matter of fact, Melatonin is probably the most powerful endogenous free radical scavenger known at present.

Besides its ability to directly neutralize a number of free radicals and reactive oxygen species, it stimulates several antioxidative enzymes which increase its efficiency as antioxidant.

In terms of direct free radical scavenging, melatonin interacts with the highly toxic hydroxyl radical with a rate constant to that of other highly efficient hydroxyl radical scavengers. Additionally, melatonin neutralizes hydrogen peroxide, singlet oxygen, peroxynitrite anion, nitric oxide and hypochlorous acid. The following antioxidant enzymes are stimulated by melatonin: superoxide dismutase, glutathione peroxidase and glutathione reductase. Melatonin can be widely used as a protective agent against a wide variety of processes and agents that damage tissues via free radical and reactive oxygen mechanisms.

Despite improved technical handling of surgical cases, postoperative infectious and non-infectious complications and related medical treatment costs continue to be a major burden for any health care system. Surgery-induced alterations of the immune and inflammatory system are well described and may play a role in the genesis of postoperative complications. It has been recognized that an exaggerated inflammatory response and with it important injury (oxidative tissue damage, also in distant organs, i.e. lungs; microvascular leakage; neutrophil-endothelium adhesion) results from tissue ischemia and the following reperfusion, particularly in visceral tissues. This exaggerated inflammatory response together with blood transfusions and/or hemorrhage contribute to a suppression of the immune system.

With respect to preoperative administration of melatonin, it has been described in British Journal od Anesthesia 1999, 82(6): 875-80, that low level dosing of oral melatonin in a sublingual fashion is an effective pre-medication prior to administering a general anesthetic.

Furthermore, US-A-6,552,064 describes the use of melatonin for induction of general anesthesia.

In J Biol Regul Homeost Agents 1995; 9: 31-33 the immune effects of presurgical treatment of low-dose interleukin-2 in combination with melatonin are described. It is concluded that this neuroimmunetherapy is caoable to neutralize surgery-induced lymphocytopenia in cancer patients.

In a review published in Best Practice & Research Clinical Endocrinology and Metabolism 2003; 17: 273-285 the clinical implications and potential uses interms of influencing the biological clock (sleep, jet lag), immune function and cancer growth are described. In addition, a description of the newly discovered free radical scavenging and antioxidant activities is included. A list of clinical situations in which melatonin has been used with beneficial effects is included and discussed.

In J Surg Res 1996; 65: 109-114 it is described that short term melatonin administration after surgery-induced hemorrhagig shock significantly improved survival in mice subjected to septic challenge.

In J Pineal Res 2002; 32: 168-172 pretreatment with melatonin was observed to reduce the volume of cerebral infarction in a rat middle cerebral artery occlusion model.

In J Pineal Res 2003; 35: 104-108 the therapeutic use of melatonin against surgically induced oxidative stress is proposed based on in vitro tests performed with erythrocytes of patients undergoing cardiopulmonary bypass operations.

As described in J Pineal Res 2003; 34: 260-264 neutrophil apoptosis is delayed following hepatectomy compared to the preoperative state. Melatonin, in vitro, can reverse this delayed process and enhances apoptosis activity in neutrophils.

In J Pediatr Surg 2004; 39: 184-189 it was found that treatment with melatonin of neonates with malformations undergoing surgery resulted in a progressive reduction of clinical parameters of inflammation. The treatment was started within 3 hours after the end of surgery.

However, non of these publications relate to or indicate the effects of melatonin on infectious postoperative complications.

It is known to treat infectious postoperative imfections by a preventive treatment with antibiotics. However, infectious postoperative complications do occur in spite of preventive treatment with antibiotics immediately before or during surgical treatment and have so far been treated upon their occurrence during the postoperative recovery period.

Additionally, some descriptions exist of approaches according to which a patient may be prepared before a planned surgical procedure by preoperative application of specific nutritional formulations in order to reduce the risk and the severity of postoperative complications.

In the patent US-A-5,656,608 a method is described for the treatment of endotoxemia by application of a therapeutically active quantity of the aminoacids glycine, alanine and serine for at least a period of three days prior to surgery.

In the patent US-A-5,731,290 a method is disclosed to improve the immune response after surgical procedures via preoperative application of a food supplement containing an immune-stimulating quantity of omega-3 fatty acids combined with L-arginine, L-ornithine or their precursors. The supplement may be given for at least three days before surgery.

WO-A-96/25,861 describes the use of glycine and glycine precursors alanine and serine for preparing a medicament or a nutritional formulation to reduce endotoxemia in patients undergoing surgical procedures. Preopeative administration is required for a period of at least 3 days before surgery.

From WO-A- 99/62,508 it is known that glycine can be used to formulate a medication for the treatment of hemorrhagic shock describing in addition the preoperative use of such a formulation.

In US-A-5,9002,829 a method is described to influence microcircualtion by preoperative administration of L-arginine, one of its precursors or other NO-donors suitable as substrate for NO-synthase. Preoperative administration occurs over a period of at least one day.

US-A-6,013,273 describes a method to treat endotoxic shock by the use of an appropriate amount of choline, which is administrated over a period of at least one day peroperatively, but normally during a period of one to six days before surgery.

Common denominator of all of the above mentioned treatments is the fact that all of them have to be initiated at least one or more days before the actual surgical procedure is carried out. However, such approaches are not feasible for treating an emergency, i.e. a trauma patient reqiring an immediate operative procedure not allowing a pretreatment time of a day or more.

In view of this prior art, the problem underlying the present invention is to provide a method that allows short term preoperative treatment of a patient - within a few hours to a few minutes - for the purpose of preventing or prophylactically or therapeutically treating postoperative infectious and non-infectious complications induced by major surgical interventions covering emergency procedures as well. Nevertheless, the present invention also covers the use of this method over a longer preoperative time period such as 24 h or more.

The solution of this problem is the use of melatonin for the manufacture of a medicament for the therapeutic treatment, prophylactic treatment and/or prevention of postoperative infectious and/or non-infections complications induced by surgical interventions covering both, elective as well as emergency procedures. The prophylactic treatment comprises both the prevention and the prophylaxis of infectious and non-infectious complications.

The invention further provides the use of melatonin alone or melatonin in combination with L-arginine or a physiologically acceptable salt thereof for the prevention and/or prophylactic treatment of postoperative complications, infectious or non-infectious ones, induced by surgery to be administered to a human being prior to surgical procedures in an amount effective to prevent and/or ameliorate surgery-induced ischemia/reperfusion injury.

The surgical intervention include preferably all operations which include an ischemia/reperfusion of an organ. Surgical interventions are defined as elective surgical procedures as well as emergency procedures. Examples of such elective surgical procedures are surgery of the upper or lower gastrointestinal tract including laparoscopic procedures, open heart surgery with or without heart/lung machine, nose and throat surgery, vascular surgery, neurological (brain) surgery, transplantations (liver, heart, lung, kidney, intestinal), surgeries on the liver and caesarean sections. Examples of emergency procedures are trauma surgery in general and surgical procedures to address septic foci.

The infectious complications are preferably selected from the group consisting of pneumonia, wound infection (wound dehiscence), intra-abdominal abscess, and urinary tract infection (UTI) and the non-infectious complication is preferably anastomotic leak.

More specifically, the prophylactic treatment according to the invention reduces the rates of occurrence of these infections and of anastomotic leaks to a considerable degree and ameliorates the severity of such infections in case they still occur.

In accordance with the invention it has more particularly been found that preoperative application of melatonin, in a dose dependent fashion, inhibits and/or ameliorates the major effects of partial liver resection in rats i.e. the elevation in liver enzymes (ALT, AST) indicating substantial liver damage induced by the surgery. Melatonin further reduces the increase in hypoxia of liver tissue observed under partial liver resection. Melatonin also reduces the extent of necrosis following ischemia/reperfusion of the liver. Thus, it is clear that preoperative application of melatonin has a major protective action on the liver damage produced by partial resection of the liver.

The amount of melatonin used is preferably 0.1 to 1000 mg/day, in particular 1 to 500 mg/day, more preferred 5 to 500 mg/day, even more preferred 5 to 100 mg/day and most preferred 50 to 100 mg/day. For use in the compositions, formulations and methods of the invention, melatonin is conveniently employed in dried (powdered) form. Melatonin may also be used in the form of concentrated solutions according to the invention. The amount of 0.1 to 1000 mg/day corresponds to a daily dose of 0.002-10mg/kg body weight (BW range 50-100kg). The amount of medicament or formulation to be administered depends to a large extent on the patients' specific requirements.

The medicament will conveniently be administered in the form of unit doses suitable for administration of the formulation 1 to 3 times per day. Where the oral formulation of the invention comprises energy sources, it is appropriate not to supply more than 100 Kcal/day. Apart from this limitation with respect to the energy supply, oral formulations of the invention for preventing and/or treating postoperative complications will conveniently be supplied in the form of drinking solutions.

Preferably, the medicament further comprises a compound selected from the group consisting of L-arginine, a physiologically acceptable salt thereof, one or more other physiologically acceptable compounds associated with the synthesis of nitric oxide and mixtures thereof, more preferred L-arginine.

Physiologically acceptable salts of L-arginine are preferably phospates, acetates, citratates, maleates, lactates, tartrates, adipates, fumarates, hydrates and the like. The physiologically acceptable compounds associated with the synthesis of nitric oxide are preferably trinitroglyceride, nitroprusside, aminoguanidine, spermine-NO, spermidine-NO and SIN 1 (3-morpholinosydnonimine).

The medicament may be administered to the patient orally, enterally, transdermally or parenterally. The oral administration route is preferred, particularly for prophylactic treatment. The medicament or formulation is conveniently administered in the form of an aqueous drinking solution or in the form of galenic formulations for oral administration. The medicament or formulation in a form suitable for oral application is accordingly preferably in aqueous or in soft gelatine capsule form. The medicament or formulation of the invention may be so formulated as to deliver mealtonin in a delayed form.

If the medicament is intended for the intravenous application, a parenteral formulation is preferred. Typical pharmacologically acceptable formulation forms for intravenous administration will further comprise pharmacologically acceptable diluents, carriers, microemulsions, pigments and/or other adjuvants well known to the skilled person to be suitable for incorporation into such formulation and optionally an antibiotic suitable for preoperative phrophylaxis.

The galenic formulations of the invention may be obtained in a manner known per se, e.g. by admixing the ingredients.

Intravenous administration of the formulations of the invention may be administered as single-shot application or as sigle-shot application in combination with antibiotics (1.5 g of third generation cephalosporins) that are routinely used for infectious prophylaxis. A second dose of melatonin or melatonin combined with antibiotics may be given if the duration of the operation is longer than four hours or with an intraoperative blood loss > 1 liter.

The medicament is preferably applied less than approximately 24 hours, more preferred less than approximately 6 hours, in particular less than approximately 3 hours, more preferred less than approximately 2 hours, most preferred less than approximately 1 hour prior to the surgical intervention for the prophylctic treatment and/or prevention of the postoperative complications. The finding that the use of melatonin less than approximately 3 to 1 hour prior to the surgical intervention has the above advantages, this use is particularily suited for application in case of an emergency, i.e. where the surgical intervention was not planned.

The invention also relates to a pharmaceutical formulation comprising melatonin and at least one compound selected from the group consisting of L-arginine, a physiologically acceptable salt thereof, one or more other physiologically acceptable compounds associated with the synthesis of nitric oxide and mixtures thereof.

The amount of melatonin in the pharmaceutical formulations is as desribed above. The amount of the at least one compound selected from the group consisting of L-arginine, a physiologically acceptable salt thereof, one or more other physiologically acceptable compounds associated with the synthesis of nitric oxide or mixtures thereof is preferably 0.1 - 1000 mg, preferably 0.5 - 500 mg per dose unit, such as per tablet, soft gel capsule or per ml drinking solution. The ratio of melatonin to the at least one compound selected from the group consisting of L-arginine, a physiologically acceptable salt thereof, one or more other physiologically acceptable compounds associated with the synthesis of nitric oxide is preferably 1:20 to 5:1.

The pharmaceutical formulation may be in the form of an oral, enteral, transdermal or parenteral formulation. The pharmaceutical formulation is or is used preferably as described in relation to the use of melatonin above.

The invention, moreover, relates to a method for the prevention and/or prophylactic treatment of postoperative infectious and non-infectious complications induced by major surgical interventions covering both, elective as well as emergency procedures, comprising administering to a human being prior to a surgical procedure a medicament comprising melatonin alone or melatonin in combination with at least one compound selected from the group consisting of L-arginine, a physiologically acceptable salt thereof, at least one other physiologically acceptable compound associated with the synthesis of nitric oxide, and mixtures thereof in an amount effective to inhibit and/or ameliorate the surgery-induced complications. The preferred embodiments are as described above in relation to the use of melatonin.

The invention is further illustrated by the following Example which is not intended in any way to limit the scope of the claimed invention.

### EXAMPLE

Ischemia and subsequent reperfusion of organs and tissues induced by clamping of blood vessels to prevent excessive bleeding, are unavoidable during surgical procedures. Such a standard iscchemia/reperfusion situation can be simulated by a partial hepatectomy in a clinically relevant rat surgical model.

In this animal model, female Sprague Dawley rats (200-220g) undergo a median laparotomy followed by clamping of the right lateral liver lobe for 60 min (ev. 75 or 90 min) resulting in a hypoxia in about 30% of the entire organ. After reperfusion of the ischemic tissue, the remaining 70% of the liver, the non-ischemic middle and left lateral liver lobes are surgically removed and the surgical incision is closed. During a 7-day follow up period the effects of ischemia/reperfusion are observed with respect to liver damage and survival.

In this rat model the operative trauma of performing a laparotomy introduces a first hypoxia in the liver. The subsequent partial clamping of the blood supply to the liver and the following partial resection of a large part of liver tissue results in additional ischemia. During the subsequent reperfusion the actual free radical-induced damage to the liver tissue occurs. This actual damage to many cells of the liver tissue in regions of the remaining parts of the organ is now determined by the release of enzymes such as liver transaminases, alkaline phosphatase and lactate dehydrogenase after various time points following partial liver resection.

Preoperative application of melatonin by gavage of a melatonin-containing aqueous solution into the stomach 2 hours before the surgery results in amelioration of the massive increase in the above mentioned enzyme activities, observed in the control animals (gavaged with a aqueous control solution not containing melatonin), in a dose dependent fashion.

Similarly, an increase in survival time after 7 days is observed as an effect of the preoperative treatment with melatonin.

These effects demonstrate the benefits of preoperative application of melatonin before major surgery in preventing injury through procedure related ischemia/reperfusion situations and indicate the potential of preventing/ameliorating the occurrence of postoperative infectious and non-infectious complications.

The use of melatonin, either alone or in combination with a compound selected from the group consisting of L-arginine, a physiologically acceptable salt thereof, one or more other physiologically acceptable compounds associated with the synthesis of nitric oxide and mixtures thereof, and the pharmaceutical formulation and the method according to the invention advantageously reduce the lenght of stay in the hospital of a patient which underwent a surgical intervention. Therewith the average treatment costs of patients undergoing operative procedures are reduced.

## Claims

1. Use of melatonin for the manufacture of a medicament for the therapeutic treatment, prophylactic treatment and/or prevention of postoperative infectious and/or non-infections complications induced by surgical interventions.

2. Use according to claim 1, wherein the infectious complications are selected from the group consisting of pneumonia, wound infection (wound dehiscence), intra-abdominal abscess, and urinary tract infection (UTI).

3. Use according to claim 1, wherein the non-infectious complication is anastomotic leak.

4. Use according to any one of the preceding claims, wherein the amount of melatonin is 0.1 to 1000 mg/day.

5. Use according to claim 4, wherein the amount of melatonin is 50 to 100 mg/day.

6. Use according to any one of the preceding claims, wherein the medicament further comprises a compound selected from the group consisting of L-arginine, a physiologically acceptable salt thereof, one or more other physiologically acceptable compounds associated with the synthesis of nitric oxide and mixtures thereof.

7. Use according to any one of the preceding claims, wherein the medicament is in the form of an aqueous solution intended for oral consumption by the patient or in the form of a parenteral formulation intended for intravenous application.

8. Use according to any one of the preceding claims, wherein the medicament is applied less than approximately 24 hours prior to the surgical intervention for the prophylactic treatment and/or prevention of the postoperative complications.

9. Use according to claim 8, wherein the medicament is applied less than approximately 3 hours prior to the surgical intervention for the prophylactic treatment and/or prevention of the postoperative complications.

10. Pharmaceutical formulation comprising melatonin and at least one compound selected from the group consisting of L-arginine, a physiologically acceptable salt thereof, one or more other physiologically acceptable compounds associated with the synthesis of nitric oxide and mixtures thereof.

11. A method for the prevention and/or prophylactic treatment of postoperative infectious and non-infectious complications induced by major surgical interventions covering both, elective as well as emergency procedures, comprising administering to a human being prior to a surgical procedure a medicament comprising melatonin alone or melatonin in combination with at least one compound selected from the group consisting of L-arginine, a physiologically acceptable salt thereof, at least one other physiologically acceptable compound associated with the synthesis of nitric oxide, and mixtures thereof in an amount effective to inhibit and/or ameliorate the surgery-induced complications.

12. The method according to claim 11, wherein the medicament of the invention is applied in combination with the routinely practised preoperative infection prevention with intravenously applied antibiotics (single-shot).
